Europäisches Patentamt

European Patent Office

Office européen des brevets

(11) Veröffentlichungsnummer: **0 251 003**
**A2**

(12) **EUROPÄISCHE PATENTANMELDUNG**

(21) Anmeldenummer: **87108570.0**

(22) Anmeldetag: **13.06.87**

(51) Int. Cl.⁴: **A61K 31/41**

(30) Priorität: **18.06.86 DE 3620433**

(43) Veröffentlichungstag der Anmeldung:
**07.01.88 Patentblatt 88/01**

(84) Benannte Vertragsstaaten:
**AT BE CH DE ES FR GB GR IT LI LU NL SE**

(71) Anmelder: **BOEHRINGER INGELHEIM KG**

**D-6507 Ingelheim am Rhein(DE)**

㊈ **AT BE CH DE ES FR GB GR IT LI LU NL SE**

(71) Anmelder: **Boehringer Ingelheim International
G.m.b.H**

**D-6507 Ingelheim am Rhein(DE)**

㊈ **GB**

(72) Erfinder: **Arndts, Dietrich, Dr.**
**Mühlstrasse 7**
**D-6531 Appenheim(DE)**
Erfinder: **Schingnitz, Günter, Dr.**
**Unter den Gärten 18**
**D-6550 Bad Kreuznach 14(DE)**
Erfinder: **Streller, Ilse, Dr.**
**Waldstrasse 16**
**D-6534 Stromberg(DE)**
Erfinder: **Walland, Alexander, Dr.**
**Wilhelm-Leuschner-Strasse 20**
**D-6507 Ingelheim am Rhein(DE)**
Erfinder: **Speck, Georg, Dr., Dipl.-Chem.**
**Rheinstrasse 13**
**D-6507 Ingelheimam Rhein(DE)**

(54) **Verwendung von 2-[N-2,6-Dichlorphenyl-N-allyl-amino]-imidazolin- (2) als cytoprotektives Mittel.**

㊗ Die Erfindung betrifft die Verwendung von 2-[N-(2,6-Dichlorphenyl)-N-allylamino]-imidazolin-(2) als cytoprotektives Mittel.

EP 0 251 003 A2

## Verwendung von 2-[N-2,6-Dichlorphenyl-N-allyl-amino]-imidazolin-(2) als cytoprotektives Mittel

Die Erfindung betrifft die Verwendung von 2-[N-2,6-Dichlorophenyl-N-allylamino]imidazolin-(2) und dessen Säureadditionssalze als Mittel zur Cytoprotektion.

Es ist bekannt, daß 2-[N-2,6-Dichlorphenyl-N-allylamino]imidazolin-(2) und dessen Säureadditionssalze wertvolle pharmakologische Eigenschaften besitzen. Die Verbindung, ihre Herstellung und Verarbeitung zu pharmazeutischen Anwendungsformen ist in der deutschen Offenlegungsschrift 19 58 201 beschrieben, wobei die analgetischen Eigenschaften der in dieser Offenlegungsschrift offenbarten Verbindungen herausgestellt wird. Aus der deutschen Offenlegungsschrift 28 31 190 ist die Verwendung von 2-[N-2,6-Dichlorphenyl-N-allylamino]imidazolin-(2) sowie dessen Säueadditionssalze als bradykardes Mittel bekannt. Die Verbindung ist in der Literatur auch unter dem Namen Alinidin bekannt.

Es wurde nun überraschenderweise gefunden, daß 2-[N-2,6-Dichlorphenyl]-N-allylamino]imidazolin-(2) wie auch dessen Säureadditionssalze eine cyotprotektive Wirkung besitzt.

Die cytoprotektive Wirkung von Alinidin wurde an isolierten Rattenherzen unter Ischämiebedingungen mit nachfolgender Reperfusion (retrograde Perfusion über die Aorta nach Langendorff) und nach Perfusion mit calciumfreier und nachfolgend calciumhaltiger Lösung (Calciumparadox, Ruigrok et al., Europ. J. Cardiol. 3, 59, 1975) untersucht.

Männliche Ratten (Chbb:Thom) wurden durch Nackenschlag getötet, das Herz rasch entnommen und nach der Methode von Langendorff unter konstantem Druck (80 cm $H_2O$) perfundiert. Messung des linksventrikulär entwickelten Druckes mit flüssigkeitsgefülltem Ballonkatheter, bei Versuchen zum Calciumparadox auch Ermittlung der spontanen Herzfrequenz aus den Druckkurven.

Bei 30 Herzen wurde der rechte Vorhof abgetrennt und das Herz mit einer bipolaren, in den rechten Ventrikel eingesteckten Elektrode mit einer Frequenz von 300/min (Impulsdauer 1 msec) elektrisch stimuliert. Nach 10-minütiger Aequilibrierungsphase wurde Alinidin (3 Konzentrationen, n jeweils = 6) gelöst in unbegastem Perfusionsmedium oder in Kontrollversuchen (n = 12) das Medium allein mittels einer Infusionspumpe herznah in das Perfusionssystem infundiert (1 ml/min). 10 min danach Beginn der Ischämiephase mit 100-facher Verminderung des Flusses und Reduzierung der Infusionsgeschwindigkeit auf 0,1 ml/min. Dauer der Ischämie 60 min, danach Reperfusion mit normalem Fluß und Beendigung der Infusion. Nach Übergang zur Ischämie sistiert die Herzaktion nach 1 - 2 min. Reperfusion bewirkt starken Anstieg der Grundspannung, die Herzaktion setzt im Mittel nach 6,8 min wieder ein, zunächst unregelmäßig, nach 17 min rascher Übergang zu reizsynchronen Kontraktionen.

Alinidin bewirkte abhängig von der Konzentration in der Ischämiephase (6,6 13,3 und 33,3 μg/ml) eine Verkürzung der Dauer unregelmäßiger Herzaktion bei Reperfusion. Nach 13,3 und 33,3 μg/ml sind die Werte mit 4,4 und 5,4 min signifikant von den Kontrollwerten (17 min) verschieden.

42 Herzen wurden nach einer 20-minütigen Aequilibrierungsphase 30 min lang mit einer calciumfreien, NaEDTA-haltigen Lösung durchströmt, danach wurde für 3 min mit Normallösung reperfundiert. Nach Versuchsende wurde das Feuchtgewicht der Herzen, nach dem Trocknen und erfolgter Auslaugung mit 0,1 n HCl der Natrium-und Kaliumgehalt durch Atomabsorptinsspektrophotometrie bestimmt. - Das Perfusat wurde in 3-min-Perioden aufgefangen, gemessen und der Gehalt an Kreatinphosphokinase (CPK), Laktatdehydrogenase (LDH) und Glutamat-Oxalacetat-Transaminase (GOT) mit dem Analysenautomaten bestimmt. Perfusion mit caliumfreier Lösung bewirkt nach 1 - 2 min Sistieren der Herzaktion, bei Reperfusion ausgeprägte Kontraktur. In der Aequilibrierungsphase betrugen die Enzymabgaben 0,65 ± 0,083 U/g . min CPK, 0,55 ± 0,073 U/g . min LDH und 0,1 ± 0,09 U/g . min GOT. Während der Reperfusion stiegen die Enzymabgaben auf das 22-bis 27-fache an.

Zusatz von 16 μg/ml Alinidin zu allen Perfusionslösungen verminderte die Herzfrequenz in der Aequilibrierungsphase signifikant auf 118 Schläge/min gegenüber 184 Schlägen/min in der Kontrollgruppe (n jeweils 21). Die Enzymverluste wurden durch Alinidin gegenüber der Kontrollgruppe signifikant vermindert. Bei Berechnung der Enzymabgabe in U/g Herz . min ergaben sich folgende Werte: CPK: Verminderung von 18,8 auf 7,3; LDH: Verminderung von 12,2 auf 5,7; GOT: Verminderung von 2,4 auf 0,8. Ebenso wurde der Elektrolytgehalt der Herzen durch Alinidin signifikant beeinflußt. In der Kontrollgruppe betrug der Natriumgehalt 835 μg/g Herz und der Kaliumgehalt 1377 μg/g Herz, während in der Alinidingruppe 648 bzw. 1809 μg/g Herz gemessen wurden.

Die Ergebnisse zeigen eine Verringerung der Zellschädigung nach Ischämie/Reperfusion und im Calciumparadox. Die Verkürzung der gefährlichen Initialphase nach Reperfusion in Ischämieversuchen sowie die Verminderung des Enzymverlustes im Calciumparadox sind Ausdruck einer cytoprotektiven, frequenzunabhängigen Wirkung.

Der myocardiale ⁴⁵Ca-Gehalt als Maß der Cytoprotection:

Eine sympathomimetsche Überstimulierung wacher Ratten mit hohen Isoprenalindosen löst disseminierte Myocardnekrosen aus. Diese Gewebeschädigung stimmt morphologisch und pathophysiolgisch weitgehend überein mit Ischämie-bedingten Myocardnekrosen (A. Fleckenstein et al. in "Calcium Entry Blockers und Tissue Protection", ed. by T. Godfraind et al., Raven Press, New York, 1985). In Korrelation zur Intensität der Herznekrose steht der massive Ca⁺⁺-Einstrom ins Myocard. Bei gleichzeitiger Verwendung eines radioaktiven Indikators (⁴⁵CaCl₂) ist die Menge der myocardialen Radioaktivität ein zuverlässiges und leicht quantifizierbares Maß der Herzläsion. Cytoprotektive Wirkstoffe inhibieren dosisabhängig die Isoprenalin-bedingten Myocardnekrosen. Mit Hilfe einer von D. Arndts beschriebenen Methode (Arzneimittelforsch. 25: 1279-1284, 1975) wurden daher an insgesamt 36 wachen Ratten die cytoprotektiven Effekte peroraler Alinidindosen untersucht.

Allen Tieren spritzte man 10 μCi/kg Körpergewicht ⁴⁵CaCl₂ i.p. Neben zwei Kontrollgruppen (Je N = 6), die entweder nur physiol. Kochsalzlösung oder 30 mg/kg Isoprenalin s.c. erhielten, gab man den übrigen 4 Tiergruppen (Je N = 6) neben Isoprenalin (30 mg/kg) steigende Dosen Anlinidin p.o. (10 mg/kg, 3 mg/kg, 1 mg/kg und 0.3 mg/kg). Nach exakt 6 Stunden wurden die Tiere im Diäthylätherrausch thorakotomiert. Nach Blutentnahme aus dem rechten Herzvorhof wurden die Herzen entnommen und ca. 50 mg des rechten Ventrikels präpariert, der in SOLUENE® lysiert wurde. Die Radioaktivität des Blutplasmas und des solubilisierten Myocards wurden mittels Liquid Scintillations Spektrometrie quantifiziert. Die myocardiale Radioaktivität wurde als prozentualer Wert der Plasmaradioaktivität pro g. Gewebe errechnet. Ermittelt wurde die perorale Alinidindosis, die die Isoprenalin-bedingte myocradiale Radiocalziumaufnahme zu 50 % inhibiert. Dieser sogenannte H₅₀-Wert betrug für Alinidin 1,6 mg/kg. Diese cytoprotektive Wirkdosis liegt im gleichen Bereich wie die der Vergleichssubstanzen Verapamil oder Metoprolol, ohne jedoch die Nebenwirkungen eines Calciumantagonisten oder ß-Blockers aufzuweisen.

## Wirkung von Alinidin im Hypoxie-Toleranztest

Der Hypoxie-Toleranztest wird grundsätzlich nach folgender Methodik durchgeführt (Hoffmeister et al., 1982 * ):

Einer Gruppe von 10 Mäusen (o, Chbl: NMRI) wird die jeweilige Dosis der Testsubstanz 3 mal appliziert und zwar 24 h, 16 h und 30 min vor Beginn des eigentlichen Tests. Gleichzeitig erhalten weitere 10, als Kontrolle dienende, Tiere das Vehikel ohne Testsubstanz. Zur Prüfung der Hypoxie-Toleranz werden die so vorbehandelten Tiere (Substanzgruppe und Kontrollgruppe) in je eine Hälfte einer durchsichtigen, in der Mitte durch eine Zwischenwand unterteilten Plexiglaskammer gesetzt. Nach einer Wartezeit von 2 min wird die Kammer luftdicht verschlossen und mit einem Gasgemisch aus 96,5 % N₂ und 3,5 % O₂ (12 min) durchströmt. 6 - 7 min später sterben die ersten Tiere. Wenn in der Kontrollgruppe nur noch 2 - 3 der 10 Tiere Lebenszeichen erkennen lassen, wird die Durchströmung beendet und der Deckel geöffnet. Ohne die Tiere zu berühren, wird noch 15 min gewartet und anschließend die Zahl der überlebenden Tiere der Substanz-und Kontrollgruppe endgültig festgestellt.

Bei den vorliegenden Versuchen wurde Alinidin in physiologischer NaCl-Lösung subcutan injiziert, die Kontrolltiere erhielten s.c. Injektionen von NaCl-Lösung.

In Abweichung vom allgemeinen Schema wurde bei den Versuchen mit intracerebraler Applikation nur eine einmalige Dosis von Alinidin, ebenfalls in NaCl-Lösung, eine halbe Stunde vor Versuchsbeginn verabreicht. Entsprechend erhielten bei diesen Experimenten auch die Kontrolltiere nur eine einmalige intracerebrale Injektion einer NaCl-Lösung.

Die Ergebnisse zeigen eine verbesserte Hypoxie-Toleranz bei Vorbehandlung mit Alinidin.

Aufgrund dieser Befunde sollen die hier beschriebenen Verbindungen bzw. ihre Säureadditonssalze als Wirkstoff für Arzneimittel mit cytoprotektiver Wirkung Verwendung finden.

Erfindungsgemäß können sie therapeutisch oder prophylaktisch bei den nachfolgend aufgeführten Indikationen als Cytoprotectivum angewendet werden:

* Hofmeister, F.; Benz, U.; Heise, A.; Krause, V.; Neuser, H.P.
Behavioral Effects of Nimodipine in Animals
Arzneim.-Forsch./Drug Reseaech 32 (I), 347-360 (1982)

Schutz von Zellen vor dem Zugrundegehen im Streß, insbesondere durch Hypoxie; Verhinderung der disseminierten Nekrose im Verlauf der koronaren Herzkrankheit und der Herzinsuffizienz, cerebrale Stoffwechselstörungen, hirnorganisches Psychosyndrom, cerebraler Sauerstoffmangel, Behandlung der Cerebralsklerose, cerebrale Apoplexie, Zellschädigungen infolge von Drogenmißbrauch, insbesondere durch Alkoholismus.

Die Verbindungen können sowohl enteral als auch parenteral verabreicht werden. Als Einzeldosis werden 1 bis 50 mg Wirkstoff vorgeschlagen. Die gewünschte Dosis ist n der Indikation und Darreichungsform abhängig und kann experimentell bestimmt werden.

Geeignete Anwendungsformen sind beispielsweise Tabletten, Kapseln, Zäpfchen, Lösungen, Säfte, Emulsionen, Aerosole oder dispersible Pulver. Entsprechende Tabletten können beispielsweise durch Mischen des Wirkstoffes mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure, hergestellt und in Injektionsfalschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol bzw. dessen Derivaten herstellen.

Beispiele

Herstellungsbeispiel für den Wirkstoff

2-[N-(2,6-Diochlorphenyl)-N-allyl-aminol]-imidazolin-(2)

2,0 g 2-(2,6-Dichlorphenylamino)-2-imidazolin werden zusammen mit 3 ml Allylbromid und 1 ml Pyridin in 10 ccm absolutem Methanol ca. 15 Stunden lang im Rohr auf 100°C erwärmt. Die Reaktionsmischung wird hierauf im Vakuum zur Trockene eingeengt und der verbleibende Rückstand in wenig verdünnter Salzsäure gelöst. Zu ihrer Reinigung wird die salzsaure Lösung mit Äther extrahiert und die Ätherextrakte verworfen. Die hierauf mit 5 n Natrolauge freigesetzte ölige Imidazolinbase kristallisiert nach einiger Zeit unter Eiskühlung durch. Sie wird abgesaugt, mit destilliertem Wasser gewaschen und getrocknet. Die Ausbeute beträgt 1,5 g, das sind 83,8 % der Theorie.

Fp. 130 - 131°C. Das auf übliche Weise hergestellte Nitrat schmilzt bei 136 - 138°C.

Andere Säureadditionssalze lassen sich nach bekannten Verfahren herstellen.

4

## Beispiel A: Tabletten

| | |
|---|---:|
| 2-[N-2,6-Dichlorphenyl-N-allylamino]imidazolin-(2)-HBr | 10 mg |
| Milchzucker | 65 mg |
| Maisstärke | 125 mg |
| sek. Calciumphosphat | 40 mg |
| lösliche Stärke | 3 mg |
| Magnesiumstearat | 3 mg |
| kolloidale Kieselsäure | 4mg |
| insgesamt | 250mg |

Herstellung:

Der Wirkstoff wird mit einem Teil der Hilfsstoffe vermischt, intensiv mit einer wäßrigen Lösung der löslichen Stärke durchgeknetet und in üblicherweise mit Hilfe eines Siebes granuliert. Das Granulat wird mit dem Rest der Hilfsstoffe vermischt und zu Dragéekernen von 250 mg Gewicht verpreßt, die dann in üblicherweise mit Hilfe von Zucker, Talkum und Gummi arabicum dragiert werden.

## Beispiel B: Ampullen

2-[N-2,6-Dichlorphenyl-N-allylamino]imidazolin-(2)-
HBr                                                              1,0 mg

Natriumchlorid                                                  18,0 mg

dest. Wasser                                      ad            2,0 ml

Herstellung:

Wirkstoff und Natriumchlorid werden in Wasser gelöst und
unter Stickstoff in Glasampullen abgefüllt.

## Beispiel C: Tropfen

2-[N-2,6-Dichlorphenyl-N-allylamino]-
imidazolin-(2)-HBr                                             0,02 g

p-Hydroxybenzoesäuremethylester                               0,07 g

p-Hydroxybenzoesäurepropylester                               0,03 g

entmineralisiertes Wasser                          ad         100 ml

**Ansprüche**

1. Verwendung von 2-[N-2,6-Dichlorphenyl-N-allylamino]-imidazolin-(2) und dessen Säureadditionssalze als Mittel zur Cytoprotektion.

2. Verwendung von 2-[N-2,6-Dichlorphenyl-N-allylamino]-imidazolin-(2) und dessen Säureadditionssalz zur Herstellung eines Arzneimittels mit cytoprotektiver Wirkung.

6